# EUROPEAN PATENT SPECIFICATION

(11) **EP 1 719 476 B1**
(45) Date of publication and mention of the grant of the patent: **23.11.2011**
(21) Application number: 05425303.4
(22) Date of filing: 06.05.2005
(51) Int. Cl.: A61F 2/24

(54) **Annuloplasty prosthesis**
Annuloplastieprothese
Prothèse pour annuloplastie

(43) Date of publication of application: 08.11.2006
(73) Proprietor: SORIN BIOMEDICA CARDIO S.R.L., 13040 Saluggia (Vercelli) (IT)
(72) Inventor: Bergamasco, Giovanni, 10137 Torino (IT); Burriesci, Gaetano, 90135 Palermo (IT); Stacchino, Carla, 10132 Torino (IT)
(74) Representative: Bosotti, Luciano

(56) References cited:
- EP-A- 1 348 406
- WO-A-2005/007037
- SU-A1- 577 022
- US-A- 5 961 539
- US-A- 6 102 945
- US-B1- 6 368 348

## Description

This invention relates in general to a device for heart valve repair surgery, in particular an annuloplasty prosthesis.

More specifically, the present invention relates to a prosthesis having the features disclosed in the preamble of claim 1. A known prosthesis of this kind is disclosed in US-A-6 102 945.

A human heart has four heart valves: the mitral valve, the tricuspid valve, the pulmonary valve and the aortic valve.

The mitral valve is located in the left atrio-ventricular opening and controls blood flow in a single direction from the atrium to the ventricle. It opens during diastole and closes again during systole, preventing blood from flowing back from the ventricle to the atrium. The annulus of a normally functioning mitral valve is characterised by shape, dimensions and flexibility such as to permit correct closure of the valve edges during the systolic stage. For example the mitral annulus has a characteristic "kidney" (or "D") shape, and is more flexible in the portion corresponding to the posterior lip of the valve. Diseases or genetic defects can cause deformation or expansion of the annulus of the mitral valve, giving rise to incomplete closure with the consequent regurgitation of blood.

The same phenomenon may occur in the tricuspid valve, which is located between the right atrium and the right ventricle.

A method which is frequently used to eliminate some pathological changes in mitral and tricuspid valves is that of restoring the valve annulus to its correct shape and size through surgical procedures known by the name of annuloplasty.

Annuloplasty comprises surgically implanting a prosthesis supporting the expanded or deformed annulus in order to restore its dimensions and/or physiological shape in such a way as to allow the heart valve to function correctly.

The support prostheses used in valve repair surgery take the name of annuloplasty prostheses. In most cases these prostheses comprise a closed or open ring structure comprising an internal element in the shape of a ring and an outer coating of biocompatible material which permits surgical suture.

Various types of annuloplasty prostheses are described in the known art. In particular, prostheses whose rigidity can be rendered variable in a desired way depending upon the point, direction and/or manner in which stresses are applied are already known. For example patent application EP-A-1 348 406 describes various embodiments of an annuloplasty prostheses comprising a laminar or tubular member having a plurality of openings in at least one portion thereof.

Typically, in order to shape a member of this kind into a ring, an operation in which a straight structure is shaped in order to confer the typical D shape upon it is first performed and then an operation to join the free ends, for example, by welding, is performed. Both these operations require that thermal and/or mechanical processes be carried out on an overall or local scale, and these can adversely affect the thermal/mechanical properties of the constituent material, which is usually an alloy, for example an alloy based on titanium, chromium-cobalt or nickel-titanium.

The object of this invention is to provide an annuloplasty prosthesis in which the ring-shaped member can be constructed by a process which does not have the abovementioned disadvantages, while nevertheless having a geometry such as to confer the required mechanical characteristics upon it, in particular yielding to stresses which are exerted in its general plane which is greater than to stresses which are exerted in directions normal or oblique to that plane.

According to the invention this object is achieved through an annuloplasty prosthesis having the features of claim 1 which follows. Preferred features of the present invention are disclosed in the dependent claims.

By "height" is meant the dimension normal to the general plane of the ring member, while by "width" is meant the dimension in the general plane of the ring member. The term "rectangular" also includes the special case in which the two dimensions in question are the same.

Preferably, the ratio between the height and width of the overall dimensions of the transverse cross-section lies between 0.5 and 5.

Advantageously, the ring-shaped member of the prosthesis according to the invention may be manufactured as a piece having a continuous structure directly from a flat sheet through the removal of material (for example by electroerosion or using machine tools), or through cutting (for example by laser). The annular continuity of the member is thus maintained through all the stages of processing, avoiding the performance of shaping and joining operations, such as welding, which could harm the thermal/mechanical properties of the constituent material. This material may for example be selected from alloys based on titanium or comprising titanium.

The surface of the ring-shaped member may be coated with a sheath of biocompatible material selected for example from the group consisting of polymers, synthetic fabrics, biological tissues and combinations thereof. The surfaces of the ring-shaped member and/or the sheath may in turn be coated with blood-compatible carbon, for example turbostratic carbon. The process for providing a coating of this material is for example described in patents US-5 084 151, US-5 387 247, US-5 370 684, US-5 133 845 and US-5 423 886 by the same applicant. This coating helps to provide better blood compatibility for the prosthesis and controlled tissue growth of the recipient body.

Further features and advantages of this invention will be apparent from the following detailed description, which is provided by way of a non-restrictive example with reference to the appended drawings, in which:
Figure 1 is a perspective view of a ring-shaped member of a prosthesis according to the invention,
Figure 2 is a plan view of a portion of the member of Figure 1,
Figure 3 is a view in cross-section along the line III-III in Figure 2,
Figures 4 to 8 are perspective views of respective alternative embodiments of the ring-shaped member according to the invention,
Figure 9 is a plan view of a portion of the member of Figure 4,
Figure 10 is a plan view of a portion of the member of Figure 5,
Figure 11 is a plan view of the member of Figure 6,
Figure 12 is a plan view of the member of Figure 7, and
Figure 13 is a plan view of the member of Figure 8.

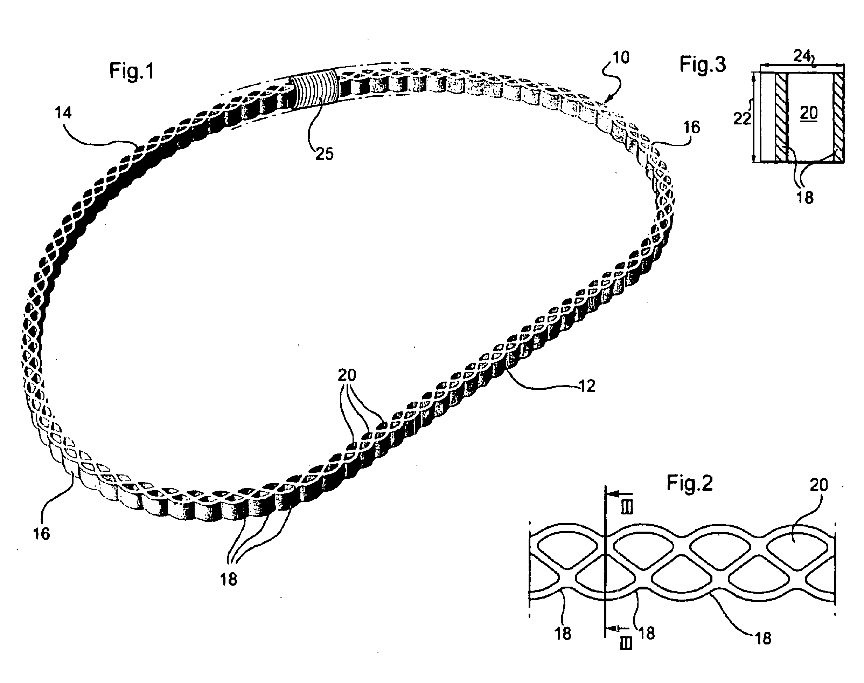

An annuloplasty prosthesis comprises (Figures 1 and 2) a member 10 of a shape generally reproducing the geometry of the annulus of a mitral valve, that is a closed ring-shape in the form of a D. The latter comprises an approximately straight intertrigonal portion 12, a first curved portion 14 opposite the straight portion and two second curved portions 16 having a more marked curvature than the first curved portion 14 connecting the latter to the extremities of the intertrigonal straight portion 12.

Ring-shaped member 10 has three walls 18 having a sinusoidal profile of the same amplitude and period and offset longitudinally with respect to each other by one third of a period in such a way that they intersect. The intersections between walls 18 form a series of openings 20 which are orientated substantially transversely with respect to the general plane of the ring-shaped member 10. In embodiments of the invention not illustrated in the figures it is possible to provide a different number of intersecting walls having a sinusoidal profile which have amplitudes, periods and longitudinal offsets which can be freely selected independently of each other.

In transverse cross-section (Figure 3), member 10 has substantially rectangular overall dimensions with a ratio between the height 22 and the width 24 which may preferably lie between 0.5 and 2.

The surface of ring-shaped member 10 is coated with a sheath of biocompatible material, of which only a portion is illustrated in Figure 1 for the purposes of clarity, and this is identified by reference number 25.

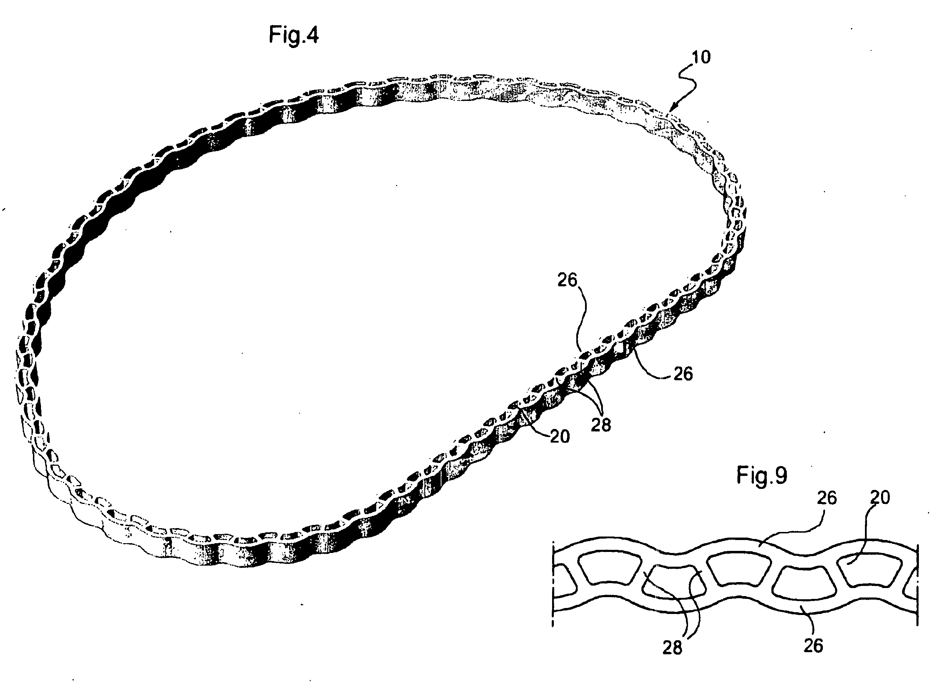

Figures 4 and 9 illustrate an alternative embodiment of the ring-shaped member in which the same numbers as are used in the preceding figures identify the same or equivalent parts.

In this case ring-shaped member 10 has a first and a second side wall 26 facing each other, which have a sinusoidal profile. The profiles of the two walls 26 match, having substantially the same amplitude and period and no longitudinal offset. In addition to this, the walls 26 are connected by interconnecting straight walls 28 at the points of flex. The member therefore has a number of openings 20 orientated substantially transversely with respect to the general plane of the ring-shaped member 10, which are separated from each other by the walls 28.

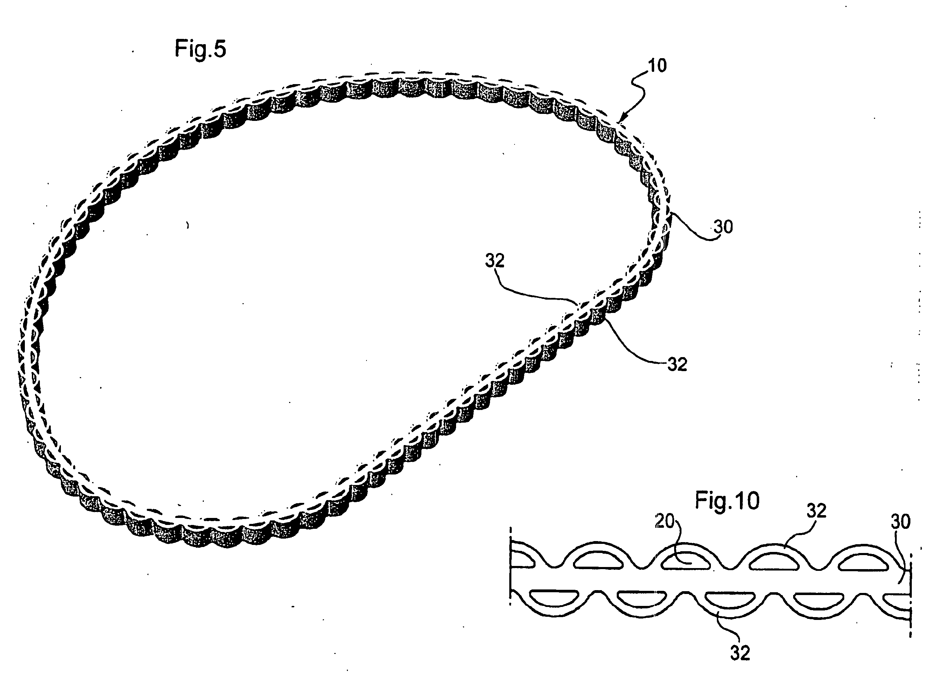

Figures 5 and 10 illustrate a further alternative embodiment of the ring-shaped member in which the same numbers as are used in the preceding figures identify the same or equivalent parts.

In this case ring-shaped member 10 comprises a continuous inner wall 30 with an outer wall 32 on each side thereof formed from a series of adjacent convex portions, the convexity of which is directed outwards. In this way, two corresponding sets of openings 20 are formed, each of these having the shape of a circular segment.

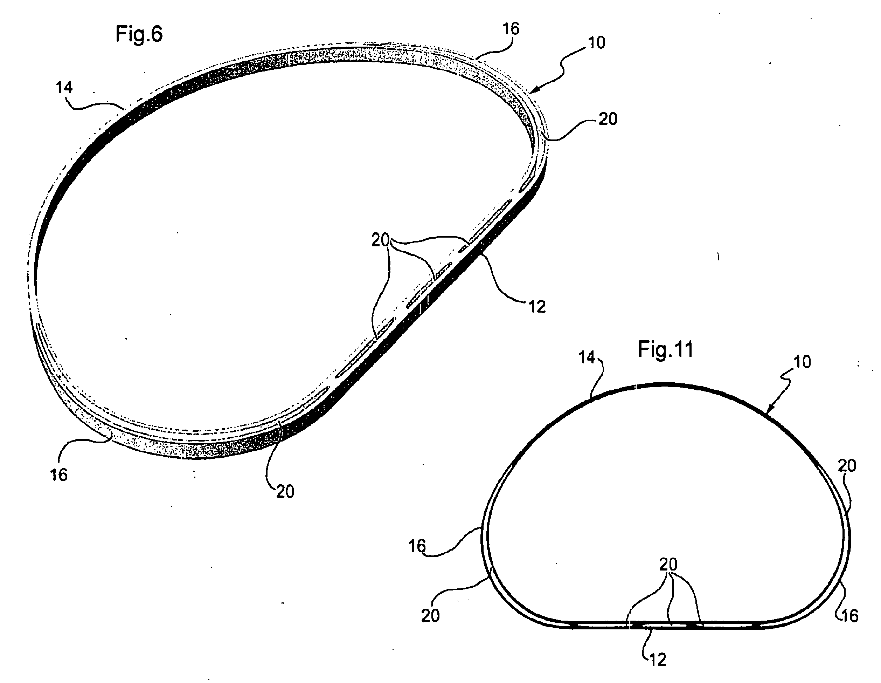

Figures 6 and 11 illustrate a further alternative embodiment of the ring-shaped member in which the same numbers as are used in the preceding figures identify the same or equivalent parts.

In this case ring-shaped member 10 has a plurality of openings 20 in correspondence of the intertrigonal portion 12 of the prosthesis and a respective opening 20 in correspondence of each curved portion 16 of more marked curvature, which connects one extremity of the intertrigonal straight portion 12 with the curved portion 14 of lesser curvature opposite to the straight portion 12. In a variant embodiment which is not illustrated in the figures, further openings 20 may be made in each curved portion 16.

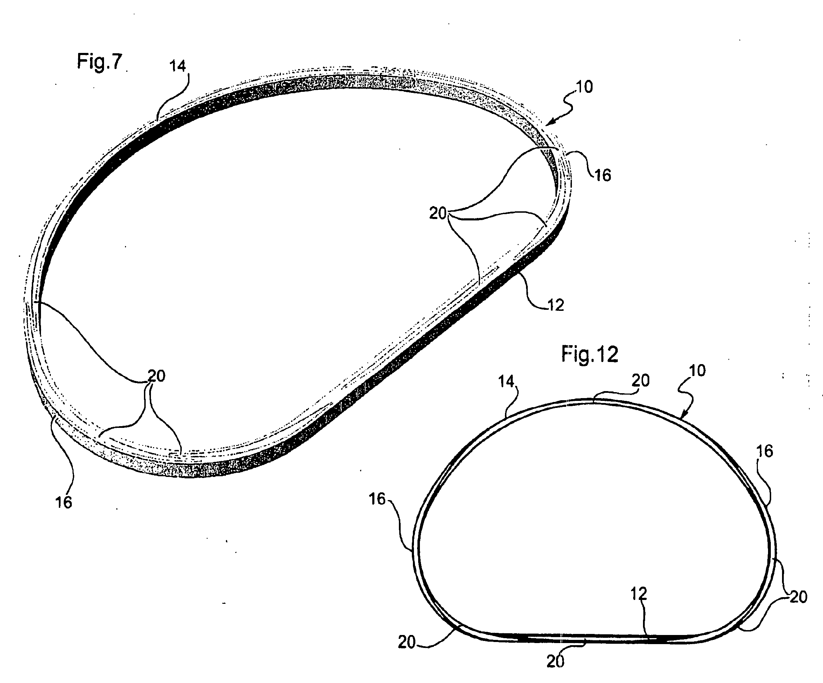

Figures 7 and 12 illustrate a further alternative embodiment of the ring-shaped member in which the same numbers as are used in the preceding figures identify the same or equivalent parts.

In this case ring-shaped member 10 has an opening 20 in the intertrigonal portion 12 of the prosthesis, a pair of openings 20 in each curved portion 16 of greater curvature and a further opening 20 in the curved portion 14 of lesser curvature.

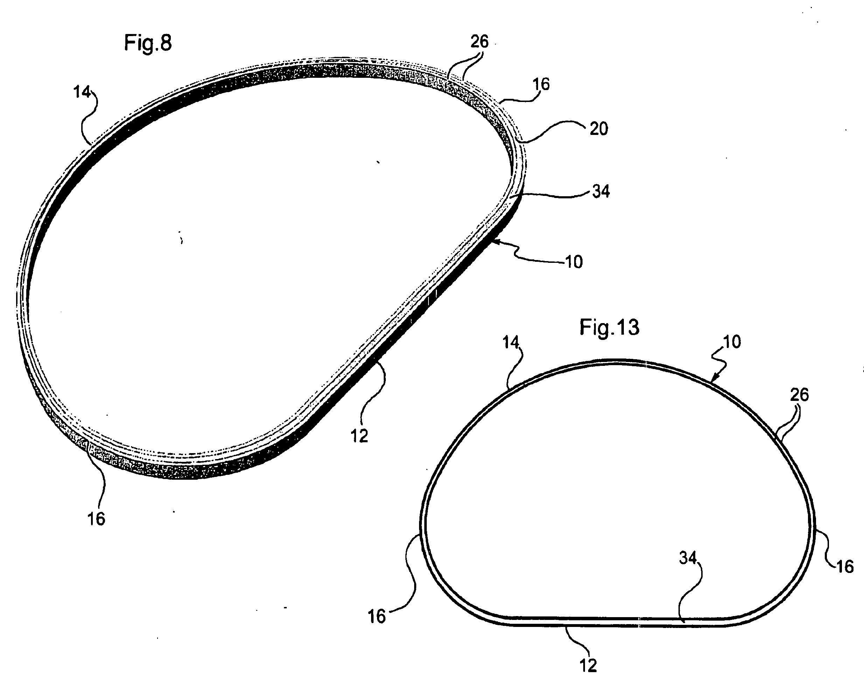

Figures 8 and 13 illustrate another alternative embodiment of the ring-shaped member in which the same numbers as are used in the preceding figures identify the same or equivalent parts.

In this case ring-shaped member 10 has a first and a second side wall 26 facing each other which bound between them a single opening 20 of annular shape. The distance between walls 26 is variable and in particular is a maximum in the intertrigonal portion 12 of the prosthesis, then decreases towards the curved portion 14 opposite to the intertrigonal portion 12 in such a way as to modulate its rigidity.

The single opening 20 is filled with elastomer material 34 which also acts as a means for connecting walls 26. Elastomer material 34 may for example be silicone, polyurethane or mixtures thereof, as described in European patent application EP-A-1 266 641 by the same applicant. In this embodiment, filler elastomer material 34 also performs the function of holding the walls together. It is however possible that such elastomer material is used to fill any of the openings in the annular member in the embodiments previously illustrated, essentially for the purpose of regulating the elastic properties of the prosthesis in an even more local manner.

Of course, without altering the principle of the invention, details of construction and embodiments may be varied widely from what has been described purely by way of example without going beyond its scope as claimed. In particular the size and spacing of the openings may remain constant over the entire extent of the ring-shaped member or may be varied so that the rigidity of the prosthesis can be controlled in a desired way depending upon the location and the direction of application of stresses. Likewise, it is in principle possible to arrange the openings in an irregular way or in a pattern which repeats regularly.

## Claims

1. Annuloplasty prosthesis comprising a ring-shaped member (10) having substantially rectangular overall dimensions of the transverse cross-section with a ratio between height (22) and width (24) of between 0.2 and 10 and at least one through opening (20) orientated substantially transversely to the general plane of the ring-shaped member (10), said prosthesis being **characterised in that** the said ring-shaped member (10) has a plurality of openings (20) formed by the intersection of a plurality of longitudinally offset walls (18) having a sinusoidal profile , or the said ring-shaped member (10) has a first and a second side wall (26) facing each other so as to bound the at least one opening (20) between them, having or matching sinusoidal profile and being connected by straight interconnecting walls (28) at the points of flex or said ring-shaped member (10) comprises a continuous inner wall (30), on each of the two sides of which there is an outer wall (32) formed from a series of adjacent convex portions whose convexity faces outwards in such a way as to form a corresponding series of openings (20) each of which has the shape of a segment of a circle.

2. Prosthesis according to Claim 1, in which the said ratio between the height (22) and width (24) of the overall dimensions of the transverse cross-section lies between 0.5 and 5.

3. Prosthesis according to any of the preceding claims, in which the said ring-shaped member (10) has a continuous structure without any joints.

4. Prosthesis according to Claim 1, in which the said ring-shaped member (10) has a plurality of openings (20) formed by the intersection of three walls (18) having sinusoidal profiles of the same amplitude and period and longitudinally offset from each other by one third of a period.

5. Prosthesis according to any of the preceding claims, in which the said ring-shaped member (10) is surrounded by a sheath (25) of a coating of biocompatible material.

6. Prosthesis according to Claim 5, in which the said biocompatible material is selected from the group consisting of polymers, synthetic fabrics, biological tissues and combinations thereof.

7. Prosthesis according to any of the preceding claims, in which at least one portion of the surface of the said member (10) and/or the said sheath (25) is coated with blood-compatible carbon.

8. Prosthesis according to Claim 7, in which the said blood-compatible carbon is turbostratic carbon.

9. Process for the production of a prosthesis according to any of the preceding claims, in which the said ring-shaped member (10) is obtained from a flat sheet by removal of material or cutting, with the annular continuity of the member (10) being maintained during all stages of processing.

10. Process according to Claim 9, in which the said removal of material is carried out by means of electroerosion or machine tools.

11. Process according to Claim 9, in which the said cutting is performed by laser.

## Patentansprüche

1. Annuloplastie-Prothese mit einem ringförmigen Element (10), das im Wesentlichen rechteckige Gesamtquerschnittsabmessungen mit einem Verhältnis zwischen Höhe (22) und Breite (24) zwischen 0,2 und 10 und wenigstens eine im Wesentlichen quer zur allgemeinen Ebene des ringförmigen Elementes (10) ausgerichtete Durchgangsöffnung (20) aufweist, wobei die Prothese **dadurch gekennzeichnet ist, dass** das ringförmige Element (10) mehrere Öffnungen (20) umfasst, die durch mehrere sich schneidende, in Längsrichtung versetzte Wände (18) mit sinusförmigem Profil gebildet sind, oder dass das ringförmige Element (10) eine erste und eine zweite Seitenwand (26) aufweist, die zueinander weisen, so dass zwischen diesen die zumindest eine Öffnung (20) angeordnet ist, und die aufeinander abgestimmte sinusförmigen Profile aufweisen und durch gerade Verbindungswände (28) an den Biegepunkten miteinander verbunden sind, oder dass das ringförmige Element (10) eine kontinuierliche Innenwand (30) aufweist, wobei an jeder der zwei Seiten der Innenwand (30) eine Außenwand (32) vorgesehen ist, die von einer Reihe von benachbarten konvexen Bereichen gebildet ist, deren Konvexität derart auswärts weist, dass eine entsprechende Reihe von Öffnungen (20) gebildet wird, die jeweils die Form eines Kreissegmentes aufweisen.

2. Prothese nach Anspruch 1, bei der das Verhältnis zwischen der Höhe (22) und der Breite (24) der Gesamtquerschnittabmessungen zwischen 0,5 und 5 liegt.

3. Prothese nach einem der vorhergehenden Ansprüche, bei der das ringförmige Element (10) eine kontinuierliche Struktur ohne Verbindungen aufweist.

4. Prothese nach Anspruch 1, bei der das ringförmige Element (10) mehrere Öffnungen (20) aufweist, die durch drei sich schneidende Wände (18) gebildet sind, wobei die drei Wände (18) sinusförmige Profile der gleichen Amplitude und Periode aufweisen und in Längsrichtung um eine 1/3 Periode versetzt zueinander angeordnet sind.

5. Prothese nach einem der vorhergehenden Ansprüche, bei der das ringförmige Element (10) von einer Hülle (25) einer Beschichtung aus biokompatiblem Material umgeben ist.

6. Prothese nach Anspruch 5, bei der das biokompatible Material ausgewählt ist aus der Gruppe bestehend aus Polymeren, synthetischen Geweben, biologischen Stoffen und Kombinationen von diesen.

7. Prothese nach einem der vorhergehenden Ansprüche, bei der wenigstens ein Bereich der Oberfläche des Elementes (10) und/oder der Hülle (25) mit blutkompatiblem Kohlenstoff beschichtet ist.

8. Prothese nach Anspruch 7, bei der der blutkompatible Kohlenstoff turbostratischer Kohlenstoff ist.

9. Verfahren zum Herstellen einer Prothese nach einem der vorhergehenden Ansprüche, bei dem das ringförmige Element (10) aus einer flachen Bahn durch Entfernen von Material oder Schneiden hergestellt wird, wobei der ringförmige Zusammenhang des Elementes (10) während sämtlicher Bearbeitungsschritte beibehalten wird.

10. Verfahren nach Anspruch 9, bei dem das Entfernen von Material mittels Elektroerosion oder einer Werkzeugmaschine durchgeführt wird.

11. Verfahren nach Anspruch 9, bei dem das Schneiden mittels Laser durchgeführt wird.

## Revendications

1. Prothèse d'annuloplastie comprenant un élément de forme annulaire (10) ayant des dimensions totales sensiblement rectangulaires de section transversale avec un rapport entre la hauteur (22) et la largeur (24) compris entre 0,2 et 10 et au moins une ouverture de passage (20) orientée de façon sensiblement transversale par rapport au plan général de l'élément de forme annulaire (10), ladite prothèse étant **caractérisée en ce que** ledit élément de forme annulaire (10) a une pluralité d'ouvertures (20) formées par l'intersection d'une pluralité de parois longitudinalement espacées (18) ayant un profil sinusoïdal, ou ledit élément de forme annulaire (10) a une première et une deuxième paroi latérale (26) se faisant mutuellement face de manière à relier au moins une ouverture (20) entre celles-ci, ayant un profil sinusoïdal correspondant et étant raccordées par des parois d'interconnexion droites (28) aux points de flexion ou ledit élément de forme annulaire (10) comprend une paroi interne continue (30), sur chacun des deux côtés comprenant une paroi externe (32) formée d'une série de parties convexes adjacentes dont la convexité est orientée vers l'extérieur de manière à former une série correspondante d'ouvertures (20) dont chacune a la forme d'un segment de cercle.

2. Prothèse selon la revendication 1, dans laquelle ledit rapport entre la hauteur (22) et la largeur (24) des dimensions totales de la section transversale est compris entre 0,5 et 5.

3. Prothèse selon l'une quelconque des revendications précédentes, dans laquelle ledit élément de forme annulaire (10) a une structure continue sans aucune jonction.

4. Prothèse selon la revendication 1, dans laquelle ledit élément de forme annulaire (10) a une pluralité d'ouvertures (20) formées par l'intersection de trois parois (18) ayant des profils sinusoïdaux de même amplitude et période et longitudinalement espacés les unes des autres par un tiers de période.

5. Prothèse selon l'une quelconque des revendications précédentes, dans laquelle ledit élément de forme annulaire (10) est entouré par une gaine (25) d'un revêtement de matériau biocompatible.

6. Prothèse selon la revendication 5, dans laquelle ledit matériau biocompatible est choisi dans le groupe constitué de polymères, de textiles synthétiques, de tissus biologiques et de combinaisons de ceux-ci.

7. Prothèse selon l'une quelconque des revendications précédentes, dans laquelle au moins une partie de la surface dudit élément (10) et/ou ladite gaine (25) est revêtue avec du carbone compatible avec le sang.

8. Prothèse selon la revendication 7, dans laquelle ledit carbone compatible avec le sang est du carbone turbostratique.

9. Procédé pour la production d'une prothèse selon l'une quelconque des revendications précédentes, dans laquelle ledit élément de forme annulaire (10) est obtenu à partir d'un feuille plate par élimination de matériau ou découpe, la continuité annulaire de l'élément (10) étant maintenue pendant tous les stades du traitement.

10. Procédé selon la revendication 9, dans lequel ladite élimination de matériau est effectuée au moyen d'une électroérosion ou de machines-outils.

11. Procédé selon la revendication 9, dans lequel ladite découpe est effectuée par laser.
